# FASCICULE DE BREVET EUROPEEN

(11) **EP 2 389 151 B1**
(45) Date de publication et mention de la délivrance du brevet: **21.04.2021**
(21) Numéro de dépôt: 10707327.2
(22) Date de dépôt: 20.01.2010
(51) Int. Cl.: A61F 13/505, A61F 13/494, A61F 13/74, A61F 13/68, A61F 13/49

(54) **STRUCTURE DE TYPE COUCHE ABSORBANTE REUTILISABLE, ET MANCHON ASSOCIE**
STRUKTUR MIT WIEDERVERWENDBARER ABSORPTIONSSCHICHT UND ZUGEHÖRIGE MUFFE
STRUCTURE WITH A REUSABLE ABSORBENT LAYER, AND ASSOCIATED SLEEVE

(30) Priorité: 20.01.2009 FR 0900240
(43) Date de publication de la demande: 30.11.2011
(60) Demande divisionnaire: 12177905.2
(73) Titulaire: Generation Plume, 75010 Paris (FR)
(72) Inventeur: HALLOUIN, Florence, 75019 Paris (FR)
(74) Mandataire: IP Trust
(86) Numéro de dépôt international: PCT/FR2010/050087
(87) Numéro de publication internationale: WO 2010/084285

(56) Documents cités:
- EP-A- 0 374 542
- WO-A-95/10992
- WO-A-2008/126438
- US-A- 5 891 122
- US-B1- 6 706 029

## Description

### DOMAINE TECHNIQUE DE L'INVENTION

L'invention se rapporte au domaine de l'hygiène corporelle externe et plus particulièrement aux structures ou articles destinés à absorber et retenir des sécrétions du corps.

Les produits visés par l'invention concernent notamment les couches pour bébés, pour enfants, les couches pour adultes incontinents et autres produits équivalents.

Dans ce domaine il existe plusieurs familles de produits: historiquement, les produits lavables existent depuis longtemps en particulier les couches ou langes constituées de grands carrés de gaze ou molleton de coton à plier en fonction de la morphologie du porteur . Les langes se ferment à l'aide d'une épingle ou autre système d'attache réversible puis ils sont généralement entourés d'une enveloppe en matière plastique imperméable. Cette solution est certes économique car réutilisable à l'infini, mais elle est très contraignante car nécessite des lavages fréquents.De plus le fait de plier un carré de tissu en coton et de l'attacher avec une épingle ou autre moyen d'attache rend ce système compliqué à placer sur le porteur et demande une certaine habilité notamment sur le corps d'un bébé qui bouge. En outre ce type de produits induit des fuites.

On connaît aussi par exemple par le document FR 2 872 028 des couches préformées présentant une forme anatomique, et qui sont élastiquées notamment au niveau des cuisses et dans le dos du porteur. Ces couches sont généralement dotées d'un système de fermeture réversible tel que des bandes auto agrippantes ou des boutons pressions. On prévoit d'insérer entre la face interne et la face externe de ces couches un élément absorbant amovible. Cette solution peut réduire le temps de séchage vis-à-vis de produits lavables réalisés en une seule pièce.Par ailleurs, bien que d'utilisation plus aisée que celle des langes, cette solution présente des inconvénients liés notamment à leur tenue et à leur étanchéité.

On connaît encore le brevet US 6 926 705 qui décrit un sous vêtement lavable formé par une poche intérieur liée à une enveloppe intermédiaire étant elle-même liée à une enveloppe extérieure étanche. La poche intérieure présente une bordure pourvue d'un élastique s'étendant sur tout ou partie de son bord intérieure, avec un recouvrement de l'élément absorbant

Un problème d'étanchéité peut résulter de la présence d'un matériau intermédiaire doux et généralement absorbant entre la poche et l'enveloppe externe qui, au contact des bords de la poche peut fuir par capillarité, ainsi qu'au niveau des coutures notamment entre la poche et le sous-vêtement. Un autre problème d'étanchéité peut résulter des mouvements entre les positions assises et debout du porteur ainsi que le poids dû au liquide contenu dans la structure, l'ensemble peut s'affaisser et des fuites peuvent se produire au niveau de l'entrejambe. Cette solution de l'art antérieur ne réalise pas un contact permanent entre l'élément intermédiaire et la peau de l'utilisateur.

On connaît également dans l'état de la technique le brevet EP374542 ainsi que la demande de brevet internationale WO95/10992. Ce dernier document divulgue une couche absorbante comprenant un élément extérieur lavable, ajustable sur le corps du porteur.

Le brevet US5891122 décrite une couche culotte complexe dont l'élément intermédiaire ne permet pas d'assurer une bonne étanchéité.

Le problème du lavage des couches et produits équivalents a été résolu de façon radicale par l'apparition de produits jetables, à usage unique. D'innombrables documents, en particulier des demandes de brevets, témoignent d'une activité soutenue dans ce domaine depuis plusieurs dizaines d'années.

Les couches ou articles jetables comportent en général une masse absorbant les liquides disposée entre un voile perméable côté corps et une feuille imperméable par exemple en polyéthylène côté extérieur. Le brevet français FR 2 578 163 divulgue un exemple de ce type de solution.

Le fait que ces produits soient à usage unique entraîne bien entendu une consommation importante et de ce fait un coût qui peut devenir pesant dans un budget familial.

Des couches jetables à bas coût ont été développées et commercialisées mais elles entraînent parfois des problèmes d'irritation de la peau des bébés et/ou de fuites.

De plus les produits jetables doivent être éliminés d'une manière ou d'une autre. S'ils sont jetés dans les poubelles, ils en accroissent naturellement le volume et leur traitement représente alors un coût et une dépense énergétique qui peut s'avérer non négligeable. Des contraintes d'ordre environnemental sont donc induites par ces produits à usage unique. Par ailleurs, si les articles, généralement à base d'ouate de cellulose, sont jetés dans des canalisations, ils doivent nécessairement être délitables afin de ne pas boucher lesdites canalisations. Cette caractéristique a en elle-même un coût.

Des solutions intermédiaires entre ces deux grandes familles sont connues.

A titre illustratif la demande de brevet WO 2008/030984 montre une couche réutilisable comprenant une couche interne perméable et une couche externe imperméable entre lesquelles il est possible d'intercaler de façon amovible un insert absorbant. Une fente ventrale est prévue à cet effet. L'insert peut lui-même être lavable, ou bien être jetable ; les couches internes et externes constituant cette couche sont réutilisables mais elles doivent nécessairement être lavées à chaque utilisation d'où un entretien particulièrement important.

Un concept proche est illustré dans la demande de brevet WO 2005/084601 qui divulgue une poche plane imperméable de forme sensiblement ovale, pouvant renfermer un élément absorbant. Cette poche est éventuellement munie de moyens permettant son attache amovible à l'intérieur d'un sous vêtement. Il s'agit ici de changer uniquement l'élément absorbant qui peut être jetable ou réutilisable.

Une telle solution semble peu fiable quant à son étanchéité notamment parce que la poche n'est pas suffisamment maintenue contre le corps de l'utilisateur.

Par « utilisateur» il faut comprendre le porteur de la structure absorbante.

### EXPOSE DE L'INVENTION

L'invention vise à remédier aux inconvénients de l'état de la technique et notamment à proposer des articles ou structures de type couches absorbantes réutilisables qui soient à la fois confortables, étanches, et simples d'utilisation. Etant totalement ou partiellement réutilisable, ce type de produits est avant tout économique et bénéfique pour l'environnement.

Pour ce faire, l'invention concerne selon son acception la plus générale une structure telle que définie par la revendication 1, ladite structure étant de type couche absorbante réutilisable, ayant un axe longitudinal XX et comprenant un élément extérieur de maintien ajustable sur le corps d'un utilisateur, un élément absorbant plat et un élément intermédiaire imperméable de maintien de l'élément absorbant, l'élément intermédiaire étant lié par des moyens d'attache à l'élément extérieur, ledit élément intermédiaire présentant sur chacune de ses longueurs une bordure élastique qui, à l'état étiré et aplati, permet de recouvrir au moins une zone longitudinale de l'élément absorbant caractérisée en ce que la dimension de l'élément extérieur mesurée selon l'axe XX et entre lesdits moyens d'attache, est inférieure à la dimension de l'élément intermédiaire mesurée selon la même direction XX, entre lesdits moyens d'attache, de sorte que la bordure élastique de l'élément intermédiaire reste en contact permanent avec la peau de l'utilisateur.

On entend au sens du présent brevet par « dimension de l'élément extérieur mesurée selon l'axe XX et entre lesdits moyens d'attache » la longueur mesurée sur ligne longitudinale médiane du tissu constituant l'élément extérieur, lorsque cet élément extérieur est mis à plat, sans les zones de resserrage au niveau des ouvertures pour le passage des cuisses. Les extrémités de cette ligne sont l'intersection entre l'axe XX longitudinal, et l'axe transversal (et donc perpendiculaire) passant par les moyens d'attache respectivement ventral et dorsal assurant la liaison entre l'élément extérieur et l'élément intermédiaire.

On entend au sens du présent brevet par « dimension de l'élément de l'élément intermédiaire mesurée selon la même direction XX » la longueur mesurée sur la ligne longitudinale médiane du tissu constituant l'élément intermédiaire, lorsque cet élément intermédiaire est mis à plat, sans élastiques. Les extrémités de cette ligne sont, comme pour la dimension de l'élément extérieur, définies par les intersections entre l'axe XX longitudinal, et l'axe transversal (et donc perpendiculaire) passant par les moyens d'attache respectivement ventral et dorsal assurant la liaison entre l'élément extérieur et l'élément intermédiaire.

On entend au sens du présent brevet par «inférieur» des différences de longueurs telles que définies aux deux paragraphes précédents suffisant pour que l'élément extérieur assure le maintien de l'élément intermédiaire d'une façon garantissant le contact permanent et continu, entre les bords latéraux de l'élément intermédiaire et la peau de l'utilisateur. Cette fonction conduit au résultat d'une bonne étanchéité de l'élément intermédiaire et d'une absence de fuite.

L'invention concerne en particulier une structure telle que définie dans la revendication 1. D'autres modes de réalisation de l'invention sont définis dans les revendications dépendantes.

Cette réalisation permet, par sa modularité, différents types d'utilisation en fonction des besoins et/ou des aspirations de l'utilisateur.

Par ailleurs, ladite bordure présente à l'état étiré et aplati, une convexité tournée vers l'axe longitudinal XX.

Cette caractéristique renforce l'étanchéité et la tenue de la structure sur le corps de l'utilisateur.

Par ailleurs, la dimension de l'élément extérieur mesurée selon l'axe XX et entre lesdits moyens d'attache est inférieure à celle de l'élément intermédiaire ; et la dimension de l'élément extérieur mesurée perpendiculairement à l'axe XX peut être inférieure à celle de l'élément intermédiaire au niveau de l'entrejambe.

Préférentiellement, la distance maximale H mesurée perpendiculairement à l'axe longitudinal XX, entre la bordure élastique et le bord longitudinal de l'élément absorbant est supérieure à 3 cm.

Ces caractéristiques dimensionnelles permettent d'une part à l'élément extérieur de s'ajuster de façon serrée sur l'élément intermédiaire et d'autre part de ne pas s'intercaler, au niveau de l'entrejambe, entre la peau de l'utilisateur et l'élément intermédiaire.

En outre, quelle que soit la position de l'utilisateur, on garde un contact avec le bord élastique de la couche quel que soit l'état (sec ou humide) de celle-ci.

Selon un autre aspect, l'élément extérieur est réutilisable, préférentiellement lavable, et il peut être en outre élastique.

En outre, l'élément absorbant peut être réutilisable. Sans sortir du cadre de l'invention, l'élément absorbant peut être jetable.

De façon préférée, les moyens d'attache de l'élément intermédiaire sur l'élément extérieur sont disposés selon la largeur de l'élément absorbant et comprennent des éléments d'attache réversibles. Cette caractéristique présente l'avantage d'une grande souplesse d'utilisation car on peut ainsi détacher temporairement ces deux éléments afin par exemple de les laver séparément, et/ou de remplacer l'un d'eux par un autre qui soit propre pour l'utilisation.

Sans sortir du cadre de l'invention, les moyens d'attache sont disposés selon la largeur de l'élément absorbant et comprennent des éléments d'attache inamovibles.

De façon plus spécifique, l'élément extérieur présente une forme générale en X et comporte un axe de symétrie X'X' qui coïncide avec l'axe longitudinal XX de la structure.

En outre, l'élément extérieur présente au moins une zone de renfort mécanique, non extensible, en contact dorsal avec l'utilisateur, symétrique vis-à-vis de l'axe XX et préférentiellement en forme de papillon.

Une particularité intéressante de l'invention consiste en ce que la structure comprend en outre un manchon jetable recouvrant au moins la surface de l'élément absorbant en contact avec la peau de l'utilisateur.

Par ailleurs, l'élément intermédiaire peut comprendre des ailettes longitudinales liées à chacun de ses bords longitudinaux élastiques. Cette caractéristique renforce l'étanchéité entre la structure et le corps notamment au niveau de l'entrejambe.

Selon un autre de ses aspects, l'invention couvre un manchon jetable destiné à envelopper un élément absorbant faisant partie d'une structure de type couche absorbante réutilisable. Comme il sera explicité plus en détail ci-après, le manchon représente une barrière pour certaines sécrétions du corps, notamment les matières fécales, mais il laisse passer les urines et autres fluides qui sont ainsi dirigés vers l'élément absorbant lui-même.

### BREVE DESCRIPTION DES FIGURES

D'autres caractéristiques, détails et avantages de l'invention ressortiront à la lecture de la description qui suit, en référence aux figures annexées, qui illustrent :
- la figure 1, une vue de dessus de la structure selon l'invention, en position d'utilisation ;
- la figure 2 une vue de dessus de l'élément intermédiaire et de l'élément absorbant selon l'invention, en position étirée ;
- la figure 3, une vue de côté de l'élément intermédiaire en position d'utilisation ;
- la figure 4 un schéma de l'élément extérieur, mis à plat ;
- la figure 5 un schéma de l'élément absorbant et de l'élément intermédiaire, mis à plat ;
- la figure 6 une coupe transversale schématique d'une structure selon l'invention, positionnée sur le corps d'un porteur;
- la figure 7, une coupe transversale schématique d'une structure selon un autre mode de réalisation de l'invention, en position sur le corps d'un porteur ;
- les figures 8 à 10 illustrent respectivement une vue de côté d'une enveloppe extérieure, d'un élément intermédiaire et d'un élément absorbant d'une structure selon un autre mode de réalisation de l'invention ;
- la figure 11 représente un premier mode de réalisation d'une découpe pour la réalisation de l'élément intermédiaire tel que représenté en figure 9 ;
- la figure 12 représente un second mode de réalisation d'une découpe pour la réalisation de l'élément intermédiaire tel que représenté en figure 9 ;
- la figure 13 représente une vue de dessus de l'enveloppe extérieure telle que représentée en figure 8.

Pour plus de clarté, les éléments identiques ou similaires sont repérés par des signes de référence identiques sur l'ensemble des figures.

### DESCRIPTION DETAILLEE D'UN MODE DE REALISATION

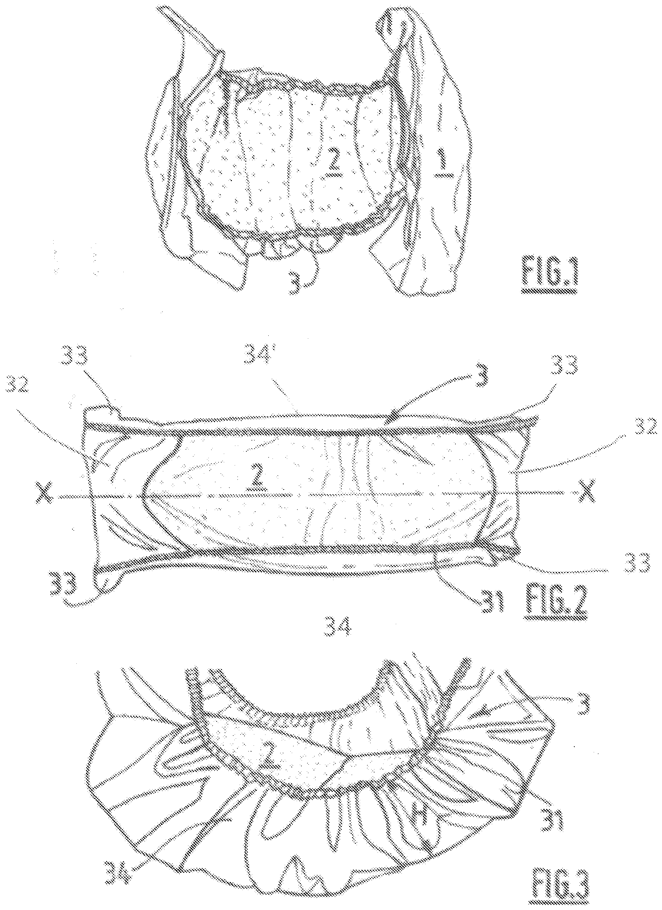

La figure 1 illustre un premier mode de réalisation d'une structure selon l'invention qui comprend notamment un élément extérieur 1 de maintien, ajustable sur le corps d'une personne. L'élément 1 est qualifié ici d'extérieur par opposition aux autres éléments constitutifs de l'invention et notamment vis-à-vis d'un élément absorbant 2 plat qui est en contact direct avec le corps de l'utilisateur. En ce sens, l'élément absorbant 2 est « intérieur » car en contact direct avec le corps de l'utilisateur. Entre ces deux éléments, on prévoit un élément intermédiaire imperméable 3 destiné d'une part à maintenir l'élément absorbant et d'autre part à assurer la liaison de l'élément absorbant avec l'élément extérieur 1.

L'élément extérieur peut être adapté sur le corps du porteur par des attaches, des boutons pressions, des éléments agrippants ou autres, disposés au niveau de la taille du porteur.

Sans sortir du cadre de l'invention, l'élément extérieur 1 peut être une culotte c'est à dire une pièce qui s'adapte directement sur la taille du porteur et comporte une ouverture pour chacune de ses cuisses.

La structure dans son ensemble présente un axe longitudinal XX.

La figure 2 montre l'élément absorbant 2 et l'élément intermédiaire 3 en position étirée, pour une meilleure compréhension de l'invention. On voit sur cette figure que l'ensemble est étiré selon sa longueur ; la longueur est définie relativement à l'élément absorbant 2 qui peut présenter une forme rectangulaire ou sensiblement rectangulaire, avec une longueur (ou grande dimension) selon l'axe XX. La matière constitutive de l'élément absorbant 2 peut être de type jetable et dégradable, par exemple à base d'ouate de cellulose. Cet aspect ne sera pas davantage décrit car à la portée de l'homme de métier, en fonction de paramètres choisis : absorption, résistance à la traction, souplesse, douceur, délitabilité. Sans sortir du cadre de l'invention, l'élément absorbant peut être réutilisable, notamment lavable.

L'élément intermédiaire 3, qui permet de maintenir l'élément absorbant 2, constitue une sorte de coque ouverte sur la plus grande surface de l'élément absorbant 2. L'élément intermédiaire 3 présente deux bordures longitudinales 31 élastiques. Tout moyen connu en soi tel qu'un ourlet garni d'un ruban élastique peut constituer ladite bordure élastique. Par ailleurs l'élément intermédiaire 3 comprend deux rabats 32 sur les deux extrémités de sa largeur, qui constituent des moyens de maintien de l'élément absorbant 2. Tout autre moyen de maintien peut être prévu sans sortir du cadre de l'invention.

L'élément intermédiaire 3 est constitué en une matière imperméable et souple que l'homme de métier choisira aisément selon ses connaissances générales.

Avantageusement les bordures longitudinales 31 de l'élément intermédiaire 3 présentent à l'état étiré (comme sur la figure 2) une convexité tournée vers l'axe longitudinal XX, de sorte que deux zones longitudinale 34, 34'recouvrent l'élément absorbant 2. Comme il sera expliqué plus en détail ci-après cette particularité améliore l'étanchéité de la structure selon l'invention.

Par ailleurs des moyens d'attache 33 sont prévus entre l'élément intermédiaire 3 et l'élément extérieur 1. Ces moyens peuvent être réversibles c'est à dire permettre une liaison temporaire ; des pressions ou encore des bandes agrippantes peuvent être prévus à cet effet. De multiples autres solutions peuvent être proposées sans sortir du cadre de l'invention.

Ainsi les moyens d'attache 33 peuvent être constitués d'une bande semi rigide fixée par exemple par couture ou par thermosoudure sur chaque largeur de l'élément intermédiaire et qui en dépasse de quelques centimètres à chaque extrémité. Les moyens d'attache 33 sont disposés à chaque extrémité longitudinale de l'élément intermédiaire 3 et à proximité de chaque extrémité de l'élément extérieur 1. Sur l'élément extérieur 1 est fixé en au moins deux points un élément souple de type ruban ou cordelette, non extensible. La distance entre les deux points est constante. Les deux points sont espacés d'une distance égale voire très légèrement supérieure à la longueur de la couture de la bande non élastique sur l'élément intermédiaire 3.Ainsi, on intercale ladite bande semi rigide entre ledit ruban et la face intérieure de l'élément extérieur 1. La bande semi rigide est en compression entre les deux points, ce qui crée une courbure. La courbure ou plus précisément la « corde » reliant les deux extrémités contribue à déformer la couche à ce niveau et à la maintenir en contact avec le corps du porteur .Par ailleurs cette déformation maintient l'élément intermédiaire 3 contre l'élément extérieur 1 lorsque cet ensemble n'est pas en place sur le corps du porteur. Les deux éléments 1 et 3 sont ainsi déformés de la même façon et leurs courbures respectives notamment dans le dos et sur le devant de la structure, sont confondues.

Selon cet arrangement, au niveau de la taille de l'utilisateur, on trouve successivement de l'extérieur vers la peau de l'utilisateur : l'élément extérieur 1, la bande semi rigide, le ruban souple et l'élément intermédiaire 3. La bande semi rigide étant ainsi intercalée, elle est plaquée contre l'utilisateur, ce qui assure un parfait maintien de l'élément intermédiaire 3. De plus ces moyens d'attache sont très faciles à mettre en place ; ils peuvent avantageusement être enlevés d'une seule main, l'autre main restant libre par exemple pour maintenir le bébé.

Selon un autre mode de réalisation, les moyens d'attache entre l'élément intermédiaire 3 et l'élément extérieur 1peuvent être des fixations définitives telles que des coutures, des soudures, de lignes de collage ...

Dans tous les cas, les moyens d'attache 33 sont disposés selon la largeur et à proximité des deux extrémités de l'élément absorbant 2 lorsqu'il est en place dans l'élément intermédiaire 3.

L'élément absorbant 2 peut en outre être constitué d'une matière réutilisable, notamment lavable. Il est ainsi possible de réutiliser à l'infini cet élément.

Par ailleurs selon un mode de réalisation, l'élément absorbant 2 étant de forme rectangulaire, il est très facile à obtenir à partir d'un simple pliage d'un tissu. Bien entendu le tissu pourra âtre choisi par l'utilisateur et/ou fourni par le fabriquant. L'utilisateur a ainsi la faculté de choisir lui-même ce qui constitue l'élément absorbant 2, que ce dernier soit jetable ou réutilisable.

La figure 3 montre de façon plus précise l'élément intermédiaire 3 avec l'élément absorbant 2 en place à l'intérieur, en position d'utilisation. On voit que chacune des zones dites longitudinales 34 présente une certaine hauteur H mesurée entre la bordure élastique 31 et le bord longitudinal de l'élément absorbant 2. La hauteur H peut varier sur la longueur de l'élément intermédiaire. De façon générale la hauteur H représente entre 8% et 20% (selon la taille du porteur) de la longueur de l'élément intermédiaire qui forme une sorte de hamac pour l'élément absorbant 2, les moyens d'attache 33 constituant les parties hautes dudit hamac.

La valeur maximale de la hauteur H est située au milieu de la longueur de la structure, mais elle peut en être légèrement décalée. Préférentiellement elle se situe au niveau de l'entrejambe.

Conformément à un mode particulier de réalisation de l'invention, l'élément intermédiaire 3 présente un pliage à chacune des extrémités de ses bords longitudinaux, ce pliage permettant de créer un soufflet au milieu de sa longueur et d'y obtenir ladite hauteur maximale H.

Selon un autre mode de réalisation de l'invention, à l'état développé, l'élément intermédiaire 3 présente une forme de tonneau dont les bords transversaux, droits, peuvent être compris entre 4 et 15 centimètres.

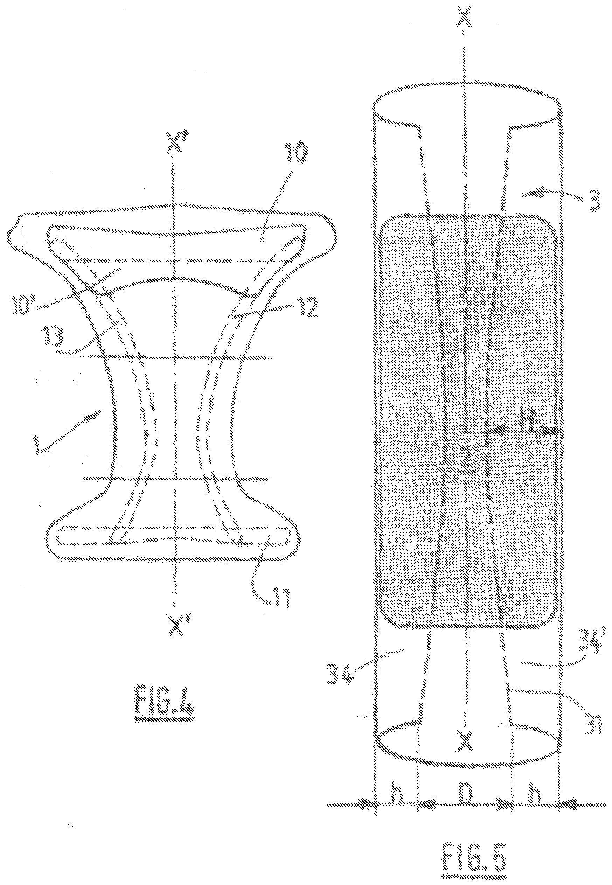

La figure 4 illustre un exemple d'élément extérieur 1 en forme générale d'X ou de sablier et qui est pourvu d'un axe de symétrie X'X' qui coïncide avec l'axe XX de la structure lorsque celle-ci est en place. L'élément 1 est réutilisable et préférablement lavable.

Selon un mode de réalisation, l'élément extérieur 1 présente des zones de renfort mécanique ici au nombre de quatre 10, 11, 12, 13. Deux de ces zones 10,11 sont sensiblement perpendiculaires à l'axe de symétrie X'X' tandis que deux autres zones 12, 13 présentent une courbure de convexité tournée vers l'axe de symétrie X'X'.

Selon un autre mode de réalisation de l'invention, l'élément extérieur 1 présente au moins une zone de renfort mécanique 10', non extensible, en contact direct avec le dos de l'utilisateur, symétrique vis-à-vis de l'axe X'X', et préférentiellement en forme de papillon. Toute forme proche, constituée de deux lobes symétriques vis-à-vis de l'axe de symétrie X'X' peut être envisagée sans sortir du cadre de l'invention. Cette caractéristique assure un bon maintien de la structure sur le corps quelle que soit la position et/ou les mouvements de l'utilisateur.

Les zones de renfort 10, 10', 11, 12, 13 peuvent être obtenues par une pièce rapportée sur l'élément extérieur 1, par exemple cousue, collée, thermocollée, soudée.On peut aussi envisager un tricotage spécifique ou encore une technique de chauffage ou thermoformage d'un tissu à base de Lycra (marque déposée) ou autres fibres synthétiques, traitement qui permet de rigidifier et/ou de déformer les zones chauffées, voire traitées par tout autre traitement de déformation. De façon connue, ce type de traitement consiste en un chauffage ou thermoformage opéré simultanément à une extension du tissu concerné, qui permet une fois le tissu refroidi, d'obtenir et de maintenir le tissu dans un état de déformation permanente. Si ces types de traitement sont réalisés alors l'élément extérieur est dépourvu de coutures.

La largeur de l'élément extérieur 1, mesurée perpendiculairement à l'axe XX' est préférentiellement inférieure à celle de l'élément intermédiaire 3, au niveau de l'entrejambe. Ceci évite de mouiller l'élément extérieur 1 qui ainsi n'est pas en contact avec le pli pubien. Avantageusement, un placage de l'élément 1 contre l'élément intermédiaire 3 est obtenu selon l'invention.

L'élément extérieur 1 peut être constitué en une matière extensible selon une ou plusieurs directions telle qu'un tissu à base de fibres naturelles de coton, ou à base de fibres synthétiques, ou encore en matière à base de fibres artificielles ; les renforts non extensibles peuvent être constitués d'une toile de coton ou d'une matière synthétique.

L'élément intermédiaire 3 peut être un tissu enduit de polyuréthane imperméabilisé. Il présente un effet déperlant sur l'une de ses faces et est imperméable sur l'autre de ses faces.

La figure 5 montre de façon très schématique l'élément intermédiaire 3 et l'élément absorbant 2 vus de dessus et mis à plat, sans les attaches 33 ni les rabats 32. Il ressort que l'élément intermédiaire 3 recouvre l'élément absorbant 2 sur deux zones longitudinales 34 et 34'de hauteur maximale H. Les limites longitudinales des zones 34 et 34'sont respectivement définies par les bords longitudinaux de l'élément absorbant 2 et les bords élastiques 31 .Si comme illustré par la figure 5, l'élément absorbant 2 est de forme rectangulaire, les bords élastiques 31 présenteront une convexité tournée vers l'axe longitudinal XX. Si l'élément absorbant 2 est en forme de sablier, les bords élastiques seront préférentiellement droits.

Dans tous les cas, en position d'utilisation, il se formera un hamac de profondeur maximale H, au milieu de sa longueur. Les bords élastiques 31 restent ainsi en contact permanent avec la peau de l'utilisateur, quels que soient les mouvements de celui-ci et quel que soit l'état de l'élément absorbant 2. Par «état», on entend le niveau d'absorption de cet élément 2. L'état va d'un niveau sec, dénué de liquide, à un état saturé où l'élément est gorgé et/ou saturé en liquide et/ou de matières fécales.

Par ailleurs, comme visible sur la figure 5, chacune des deux extrémités transversales de l'élément 3 présente une longueur égale à : D + 4xh ; h étant la demi largeur de recouvrement de l'élément 3 sur l'élément 2, et D + 2xh étant la largeur maximale de l'élément absorbant 2.

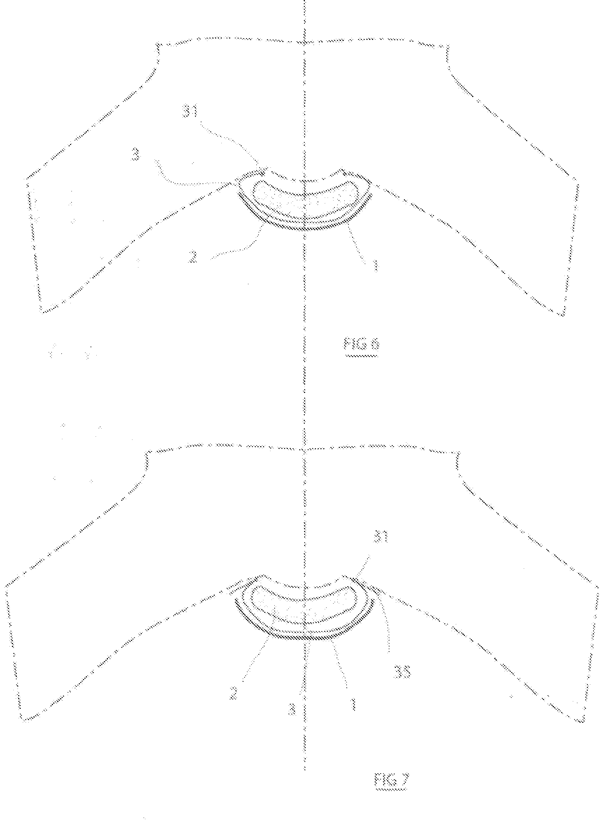

La figure 6 illustre par une coupe transversale schématique une structure selon l'invention en place sous la zone pubienne d'une personne.

On y voit l'élément extérieur de maintien 1, l'élément intermédiaire ou hamac imperméable 3, et l'élément absorbant 2 au plus proche de la zone pubienne. Les bordures élastiques 31 se trouvent insérées et calées dans le pli pubien permettant ainsi une étanchéité dans cette zone. En dehors de cette zone c'est à dire au niveau de l'aine et de l'extérieur des cuisses au niveau du bas des fesses, les bordures 31 restent en contact permanent avec la peau de l'utilisateur. Ceci est dû notamment aux formes spécifiques et conjuguées desdites bordures 31 et de l'élément absorbant 2, telles qu'explicitées ci-avant.

Ainsi, l'élément intermédiaire 3 n'est pas suspendu « librement » puisqu'il est maintenu en permanence contre le porteur.

La figure 7 illustre un autre mode de réalisation de l'invention qui diffère de celui qui vient d'être décrit par la présence additionnelle d'ailettes longitudinales 35 issues de chacune des bordures élastiques 31 et qui en dépassent extérieurement. Les ailettes 35 permettent d'éloigner l'humidité résiduelle vis-à-vis de l'élément extérieur 1 de la structure. Elles en renforcent donc l'étanchéité. Comme il apparaît sur la figure 7, les ailettes 35 sont plaquées contre la peau ce qui permet de mieux répartir l'humidité et donc d'améliorer l'évaporation.

En outre l'invention concerne un manchon destiné à recouvrir tout ou partie d'un élément absorbant 2 qui peut faire partie d'une structure telle que décrite ci-avant.

Le manchon peut être un non tissé, dégradable et/ou délitable. Il est jetable.

Il est destiné à retenir les sécrétions non fluides du corps et à laisser passer les autres sécrétions. Sa porosité notamment déterminera le type et la limite entre ces types de sécrétions.

Ainsi il est possible, en détachant le manchon du reste de la structure, d'extraire les sécrétions non fluides telles que les matières fécales, et de jeter cet ensemble dans les toilettes par exemple. L'élément absorbant 2 peut alors être à nouveau enveloppé d'un manchon neuf et/ou lavé séparément. Le manchon évite le contact des matières fécales avec l'élément absorbant 2 qui doit cependant être lavé entre chaque change. En l'absence de manchon, il serait nécessaire de séparer les matières fécales adhérant à l'élément absorbant dans un premier temps (intervention peu appréciée des utilisateurs), avant d'envisager d'introduire l'élément absorbant dans une machine à laver le linge. Cette étape non seulement induit une manipulation peu agréable voire rédhibitoire pour l'utilisateur, mais en outre elle nécessite une consommation d'eau supplémentaire.

Selon l'invention le manchon est jetable dans les toilettes et /ou dans une poubelle tandis que l'élément absorbant peut être introduit directement dans une machine à laver, parmi tout autre linge sale. Les manipulations d'une telle structure absorbante sont donc réduites au minimum et au plus propre.

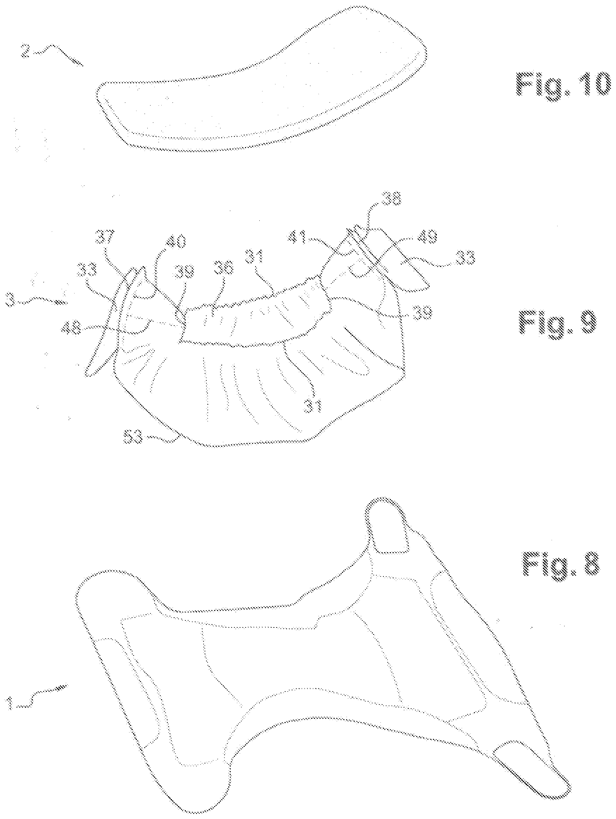

La description qui suit concerne un mode de réalisation préféré, ainsi que différentes variantes.
La couche est constituée :
- d'une enveloppe extérieure (1), représenté en figure 8, généralement réalisée en tissu, et de préférence de tissu avec des fibres élastiques, par exemple de l'élasthanne, un dérivé de polyuréthane. Cette enveloppe extérieure (1) est réutilisable et lavable
- d'un élément intermédiaire (3), représenté en figure 9, généralement réalisé en un textile imperméable ou imperméabilisé par une enduction polyuréthane, tel que du Gortex (marque déposée). Cet élément intermédiaire est également, de préférence, réutilisable et lavable. Le tissu de l'élément intermédiaire présente une caractéristique poids total (tissu + enduction) d'environ 105g/m2 avec +- 30g/m2.
- d'un élément absorbant (2), représenté en figure 10, soit jetable à usage unique, par exemple une garniture composée principalement de cellulose, ou lavable et réutilisable, en un tissu absorbant tel que du chanvre, coton, bambou, et/ou fibre synthétique.

L'élément intermédiaire (3) forme une sorte de poche ou de hamac, fixé à chaque extrémité transversale sur l'élément extérieur (1). Cet élément intermédiaire (3) contient l'élément absorbant (2) et assure l'étanchéité.

Au repos, l'élément intermédiaire (3) représenté en figure 9 présente une poche (53) dont l'ouverture (36) présente une section transversale plus petite que la section transversale de la poche dans sa plus grande largeur.

En particulier, la largeur de l'ouverture (36) selon un axe transversal est inférieure à la largeur de la poche (53) au repos, mesurée dans le plan de section où la poche est la plus enflée.

De même, la longueur de l'ouverture (36) selon un axe transversal est inférieure à la largeur de la poche (53) au repos, mesurée dans le plan de section où la poche est la plus enflée.

On entend par « au repos » la situation dans laquelle la poche est maintenue par ses deux extrémités transversales 37, 38, selon que l'on n'exerce pas de traction selon un axe longitudinal.

Au repos, l'ouverture (36) présente, vue de dessus, une forme de rectangle, avec deux bords longitudinaux (31) et deux bords transversaux (39) sensiblement rectilignes. La poche (53) présente une forme de tonneau tronqué selon une coupe longitudinale. Le tissu formant la poche est froncé le long des deux bords longitudinaux, à bords francs, sans couture ni ourlet ni repliement. Le fronçage est formé par exemple avec une machine à coudre comportant une fonction de point droit, avec un fil élastique introduit dans la cannette pour former une couture élastique.

Le fronçage peut être remplacé par un ourlet à l'intérieur duquel est placé un élastique maintenu à ses deux extrémités.

Le taux de déformation (ou « l'allongement relatif ») de l'élastique sont supérieurs à 100%, avantageusement de 120%. En d'autres termes, lorsque l'on exerce une traction sur les extrémités du bord longitudinal (31), on double la longueur par rapport à la dimension au repos. L'allongement est vérifié sous contrainte, une fois que l'élastique est mis en place sur l'ouverture (36). Cette élasticité peut être obtenu en cousant un, deux voire trois rangées d'élastiques.

L'extension maximale est obtenue lorsque l'on exerce un effort équivalent à une force exercée par un poids de 200 à 250 grammes environ.

Les bords transversaux (39) ne comportent ni fronces ni élastique.

La découpe du tissu présente deux parties convexes comme représenté en figure 5. Lorsque la poche est mise en forme, cette configuration se traduit par la forme renflée de la poche.

L'élément intermédiaire (3) présente par ailleurs deux soudures ou coutures étanches (40, 41). Ces coutures (40, 41) peuvent être réalisées par thermosoudure, soudure ultrason ou tout autre moyen équivalent empêchant la diffusion vers l'extérieur de l'élément intermédiaire d'humidité ou de liquide.

L'élément intermédiaire (3) est prolongé au-delà de ces coutures étanches (40, 41) par des attaches (37, 38).

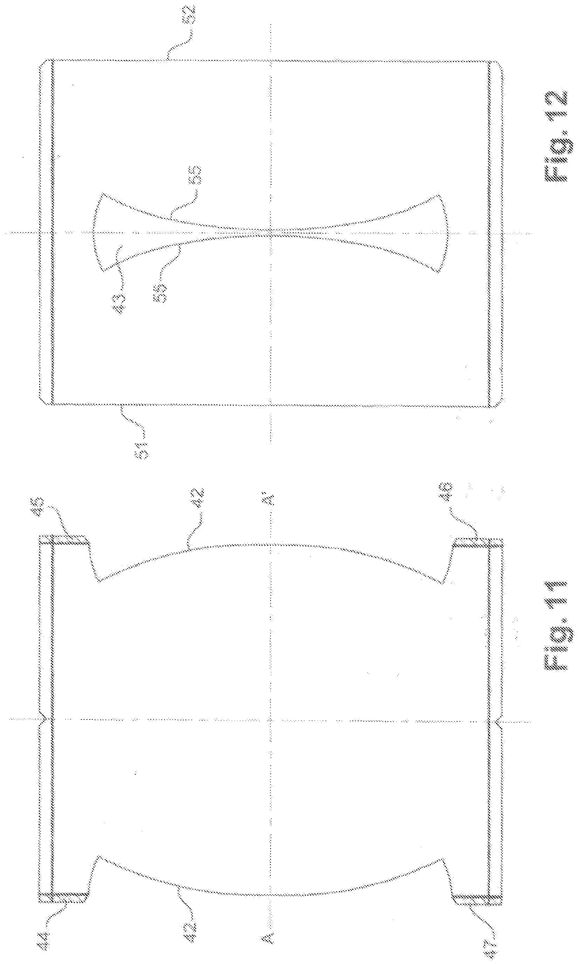

L'élément intermédiaire (3) est réalisé à partir d'une découpe dont les figures 11 et 12 représentent deux alternatives.

Le matériau utilisé pour l'élément intermédiaire (3) est un tissu synthétique à base de thermoplastique, par exemple du polyuréthane, permettant l'assemblage par friction ultrason ou par fusion, avec une maille polyamide ou polyester, enduite de polyuréthane.

La figure 11 montre une première alternative de découpe. La découpe est sensiblement rectangulaire. Le coté longitudinal présente un bord concave (42) symétrique dans l'exemple décrit par rapport à l'axe transversal médian (AA'), avec un rayon de courbure croissant de part et d'autre en s'éloignant de l'axe médian. La longueur du bord concave (42) est comprise entre 65% et 90% de la longueur totale et de 78% dans l'exemple décrit. La largeur minimale de la découpe est de 79% de la largeur maximale de la découpe.

L'élément intermédiaire (3) est formé par repliement de cette découpe et couture étanche ou soudure étanche longitudinale (48, 49) des deux pattes respectivement (44, 45) et (46, 47) longitudinales pour former une poche. Cette solution améliore l'étanchéité.

La figure 12 représente une alternative de réalisation, différent de la précédente par le fait que la découpe présente un évidemment central (43) avec deux bords longitudinaux (55) incurvés symétriquement par rapport à un axe transversal médian. La largeur maximale de cet évidemment est de 11% de la largeur, et La longueur de l'évidemment central (43) est comprise entre 65% et 90% de la longueur totale et de 78% dans l'exemple décrit.

L'élément intermédiaire (3) est formé par repliement de cette découpe et couture étanche ou soudure étanche longitudinale des deux bords longitudinaux (51, 52) longitudinales pour former une poche. Cette solution évite qu'une couture se traduisant par une légère surépaisseur ne vienne en contact avec la peau et ne crée des éventuelles irritations.

Cet élément intermédiaire (3) est fixé sur l'élément extérieur (1) par l'intermédiaire des moyens d'attache (33). Ces attaches sont formées d'une bande semi-rigide, déformable de manière non élastique, non extensible, cousue ou soudée à l'extrémité transversale (37, 38) de la poche. La bande peut être réalisée par une superposition de plusieurs textiles ou une bande thermoplastique formée avec un profil lui confèrent une semi-rigidité suffisante pour assurer l'accrochage dans une lanière prévue sur l'élément extérieur, mais limitée pour ne pas provoquer de gêne pour le porteur de la couche et pour ne pas rendre difficile le décrochage lors du remplacement de l'élément intérieur.

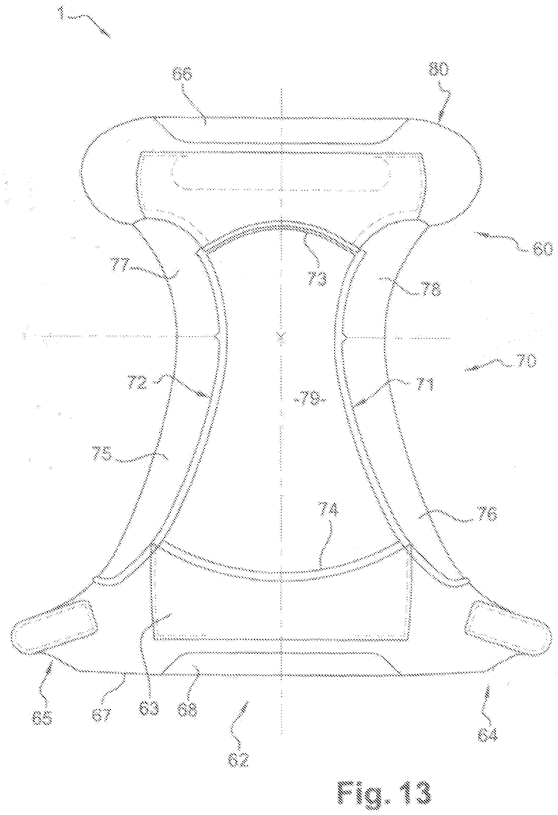

La figure 13 représente une vue de l'élément extérieur. Cet élément est dissymétrique par rapport au plan transversal, et présente une partie ventrale et une partie dorsale. Il est symétrique par rapport à l'axe longitudinal.

La forme générale est celle d'un bilboquet.

La partie ventrale (60) est non extensible ou peu extensible, afin d'éviter un affaissement autour de la ceinture abdominale du porteur lorsqu'il bouge. Dans l'exemple décrit, la bordure transversale (80) présente un empiècement (66) souple et extensible pour améliorer le confort. Cet empiècement présente une largeur d'environ deux tiers de la largeur, et une profondeur d'environ 3 centimètres.

La partie dorsale (62) présente une zone centrale (63) non extensible prolongée de part et d'autre par deux zones latérales (64, 65) qui peuvent être légèrement extensibles.

Dans l'exemple décrit, la bordure transversale (67) présente un empiècement (68) souple et extensible pour améliorer le confort. Cet empiècement présente une largeur et une profondeur sensiblement identique à celles de l'empiècement (66) de la partie ventrale.

La partie d'entrejambe (70) est formée par une pièce de tissu extensible, présentant deux bords longitudinaux (71, 72) concaves et un bord transversal (73) convexe du coté ventral.

La partie d'entrejambe présente de chaque coté de la partie ventrale une zone souple non froncé (77, 78), sans resserrage sur la cuisse lorsque la couche est portée.

Par contre, la zone souple (75, 76) du coté dorsal est froncée sur les bords (72, 71) pour rapprocher l'élément extérieur (1) de la cuisse du porteur.

La largeur cumulée des zones souples (75 à 78) mesurée au niveau de l'axe transversal médian est comprise entre un tiers et la moitié de la largeur de la zone médiane (79) à sa partie la plus rétrécie.

Ces zones souples (75, 76) sont cousues sous extension sur la zone médiane (79), afin de créer une fronce assurant un rabat de l'élément intermédiaire sur le porteur.

Du coté dorsal, le bord transversal (74) est concave pour les couches pour nouveaux-nés, et droit ou convexe pour les autres applications, afin de favoriser les possibilités d'étirement, lors des mouvements de l'utilisateur.

Une pièce de doublure non extensible peut être prévue pour renforcer la partie dorsale. Ce renforcement peut être également être obtenu par mailles bloquées en cas de fabrication par tricotage 3D, technique de fabrication appelée «seamless» ou «sans-couture».

La partie ventrale (60) et la partie dorsale (62) forme, lorsque la couche est en place, une ceinture abdominale assurant un bon maintien sur le corps du porteur. La partie d'entrejambe est suspendue par rapport à cette ceinture abdominale, et présente une élasticité qui permet de former un filet de rétention de l'élément intermédiaire. La profondeur de cette partie d'entrejambe est inférieure à la profondeur au repos de la poche (53) de la partie intermédiaire, afin de conserver le contact entre l'élément intermédiaire (3) et le corps du porteur et éviter que le tissu de l'élément extérieur ne vienne en contact avec le corps du porteur.

Selon une variante de réalisation de l'invention, qui diffère de celle décrite précédemment en référence à la figure 7, l'élément intermédiaire (3) peut comporter des ailettes imperméables additionnelles transversales, disposée longitudinalement de part et d'autre des zones de renflement (34, 34'). Chacune est assemblée longitudinalement sur les zones de renflement (34, 34') par une soudure étanche. Ces ailettes sont froncées par un élastique fixé selon l'axe longitudinal de chaque ailette. Ces ailettes froncées ramènent les bords latéraux de la poche (53) en contact avec la peau du porteur au niveau de l'entrejambe, elles empêchent les bords latéraux des zones souple (75 à 78) de l'enveloppe extérieure 1 de rentrer en contact avec les bordures élastiques 31 de l'élément intermédiaire 3 au niveau de l'entrejambe. Ces ailettes froncées permettent d'éloigner l'humidité résiduelle vis-à-vis de l'élément extérieur 1 de la structure.

Selon un autre mode de réalisation de l'invention, l'élément absorbant (2) peut être fixé par ses deux extrémités à l'élément intermédiaire (3). Une tel mode de réalisation a pour avantage de ne pas à avoir à construire la couche avant de la poser sur le porteur, entraînant ainsi un gain de temps important très appréciable, par exemple par le personnel de crèche ou de maternité.

Cette liaison se fait par le biais de 2 bandes de tissu reliant chaque extrémité de l'élément absorbant (2) à l'élément intermédiaire (3). Ces deux bandes de liaisons, de forme rectangulaire, sont en tissu imperméable. Elles ont pour longueur la largeur des extrémités de l'élément absorbant(2). Elles sont fixées à chaque extrémité de l'élément absorbant (2) par une couture selon un de leur bord longitudinal. Leur bord longitudinal opposé est soudé à l'intérieur de la poche (53) de l'élément intermédiaire (3) suivant les soudures étanches (40, 41), ou par une nouvelle soudure étanche parallèle aux soudures 40 et 41. Ces bandes permettent ainsi de relier avantageusement les 2 éléments (2) et (3) sans risque de diffusion vers l'extérieur de l'élément intermédiaire d'humidité ou de liquide.

## Revendications

1. Structure de type couche absorbante réutilisable, ayant un axe longitudinal XX et comprenant un élément extérieur de maintien (1) ajustable sur le corps d'un utilisateur, un élément absorbant (2) plat et un élément intermédiaire imperméable (3) de maintien de l'élément absorbant (2), l'élément intermédiaire étant lié par des moyens d'attache (33) à l'élément extérieur (1), ledit élément intermédiaire (3) présentant sur chacune de ses longueurs une bordure élastique (31) qui, à l'état étiré et aplati, permet de recouvrir au moins une zone longitudinale (34,34') de l'élément absorbant (2) **caractérisée en ce que** la dimension de l'élément extérieur (1) mesurée selon l'axe XX et entre lesdits moyens d'attache (33), est inférieure à la dimension de l'élément intermédiaire (3) mesurée selon la même direction XX, entre lesdits moyens d'attache (33), de sorte que la bordure élastique de l'élément intermédiaire reste en contact permanent avec la peau de l'utilisateur.

2. Structure selon la revendication 1 **caractérisée en ce que** la dimension de l'élément extérieur (1) mesurée perpendiculairement à l'axe longitudinal XX est choisie inférieure à la dimension de l'élément intermédiaire (3) au niveau de l'entrejambe du porteur de sorte que ledit élément extérieur (1) ne soit pas en contact avec le pli pubien du porteur.

3. Structure selon l'une des revendications précédentes, **caractérisée en ce que** ladite bordure (31) présente à l'état étiré et aplati, une convexité tournée vers l'axe longitudinal XX.

4. Structure selon l'une quelconque des revendications précédentes **caractérisée en ce que** la distance maximale H mesurée perpendiculairement à l'axe longitudinal XX, entre la bordure élastique (31) et le bord longitudinal de l'élément absorbant est supérieure à 3 cm.

5. Structure selon l'une quelconque des revendications précédentes **caractérisée en ce que** l'élément extérieur (1) est réutilisable, préférentiellement lavable et élastique.

6. Structure selon l'une quelconque des revendications précédentes **caractérisée en ce que** l'élément absorbant (2) est réutilisable, préférentiellement lavable.

7. Structure selon l'une quelconque des revendications précédentes **caractérisée en ce que** les moyens d'attache (33) sont disposés selon la largeur de l'élément absorbant (2) et comprennent des éléments d'attache réversibles.

8. Structure selon l'une quelconque des revendications 1 à 6 **caractérisée en ce que** les moyens d'attache (33) sont disposés selon la largeur de l'élément absorbant et comprennent des éléments d'attache inamovibles.

9. Structure selon l'une quelconque des revendications précédentes **caractérisée en ce que** l'élément extérieur (1) présente une forme générale en X et comporte un axe de symétrie X'X' qui coïncide avec l'axe longitudinal XX .

10. Structure selon l'une quelconque des revendications 1 à 9 **caractérisée en ce que** l'élément extérieur (1) présente au moins une zone de renfort mécanique, non extensible, en contact dorsal avec l'utilisateur, symétrique vis-à-vis de l'axe XX,et en forme de papillon.

11. Structure selon l'une quelconque des revendications précédentes **caractérisée en ce qu'**il comprend en outre un manchon jetable, recouvrant au moins la surface de l'élément absorbant (2) en contact avec la peau de l'utilisateur.

12. Structure selon l'une quelconque des revendications précédentes **caractérisée en ce que** l'élément intermédiaire (3) comprend en outre des ailettes longitudinales (35) liées à chacun de ses bords longitudinaux élastiques (31).

## Patentansprüche

1. Struktur vom Typ einer wiederverwendbaren absorbierenden Schicht, die eine Längsachse XX aufweist und umfassend ein äußeres Stützelement (1), das am Körper eines Benutzers anpassbar ist, ein flaches Absorptionselement (2) und ein undurchlässiges Zwischenelement (3) zum Stützen des Absorptionselements (2), wobei das Zwischenelement durch Befestigungsmittel (33) mit dem äußeren Element (1) verbunden ist, wobei das Zwischenelement (3) über jede seiner Längen einen elastischen Rand (31) aufweist, der es im gestreckten und abgeflachten Zustand ermöglicht, mindestens einen länglichen Bereich (34, 34') des Absorptionselements (2) zu bedecken, **dadurch gekennzeichnet, dass** die Abmessung des äußeren Elements (1), gemessen entlang der Achse XX und zwischen den Befestigungsmitteln (33), kleiner ist als die Abmessung des Zwischenelements (3), gemessen entlang der gleichen Richtung XX und zwischen den Befestigungsmitteln (33), so dass der elastische Rand des Zwischenelements in ständigem Kontakt mit der Haut des Benutzers bleibt.

2. Struktur nach Anspruch 1, **dadurch gekennzeichnet, dass** die Abmessung des äußeren Elements (1), gemessen senkrecht zur Längsachse XX, kleiner gewählt ist als die Abmessung des Zwischenelements (3) im Schritt des Trägers, so dass das äußere Element (1) nicht in Kontakt mit der Schamfalte des Trägers ist.

3. Struktur nach einem der vorhergehenden Ansprüche, **dadurch gekennzeichnet, dass** der Rand (31) im gestreckten und abgeflachten Zustand eine Ausbuchtung aufweist, die der Längsachse XX zugewandt ist.

4. Struktur nach einem der vorhergehenden Ansprüche, **dadurch gekennzeichnet, dass** der maximale Abstand H, gemessen senkrecht zur Längsachse XX, zwischen dem elastischen Rand (31) und der Längskante des absorbierenden Elements größer als 3 cm ist.

5. Struktur nach einem der vorhergehenden Ansprüche, **dadurch gekennzeichnet, dass** das äußere Element (1) wiederverwendbar, vorzugsweise waschbar und elastisch ist.

6. Struktur nach einem der vorhergehenden Ansprüche, **dadurch gekennzeichnet, dass** das Absorptionselement (2) wiederverwendbar, vorzugsweise waschbar ist.

7. Struktur nach einem der vorhergehenden Ansprüche, **dadurch gekennzeichnet, dass** die Befestigungsmittel (33) entlang der Breite des Absorptionselements (2) angeordnet sind und reversible Befestigungselemente umfassen.

8. Struktur nach einem der Ansprüche 1 bis 6, **dadurch gekennzeichnet, dass** die Befestigungsmittel (33) entlang der Breite des Absorptionselements angeordnet sind und nicht abnehmbare Befestigungselemente umfassen.

9. Struktur nach einem der vorhergehenden Ansprüche, **dadurch gekennzeichnet, daß** das äußere Element (1) eine allgemeine X-Form hat und eine Symmetrieachse X'X' aufweist, die mit der Längsachse XX zusammenfällt.

10. Struktur nach einem der Ansprüche 1 bis 9, **dadurch gekennzeichnet, dass** das äußere Element (1) mindestens eine mechanische, nicht dehnbare Verstärkungszone aufweist, die in dorsalem Kontakt mit dem Benutzer steht, symmetrisch in Bezug auf die Achse XX ist und eine Schmetterlingsform hat.

11. Struktur nach einem der vorhergehenden Ansprüche, **dadurch gekennzeichnet, dass** sie ferner eine Einweghülle umfasst, die mindestens die Oberfläche des Absorptionselements (2) in Kontakt mit der Haut des Benutzers bedeckt.

12. Struktur nach einem der vorhergehenden Ansprüche, **dadurch gekennzeichnet, dass** das Zwischenelement (3) ferner Längsrippen (35) aufweist, die mit jeder seiner elastischen Längskanten (31) verbunden sind.

## Claims

1. Reusable absorbent layer type structure, having a longitudinal XX axis and comprising an outer support element (1) adjustable on the body of a user, a flat absorbent element (2) and an impermeable intermediate element (3) for the retention of the absorbent element (2), the intermediate element being linked by attachment means (33) to the outer element (1), said intermediate element (3) having on each of its lengths an elastic border (31) which, in the stretched and flattened state, makes it possible to cover at least one longitudinal zone (34,34') of the absorbent element (2) **characterized in that** the dimension of the outer element (1) measured along the XX axis and between said attachment means (33), is less than the dimension of the intermediate element (3) measured in the same direction XX, between said attachment means (33), so that the elastic edge of the intermediate element remains in permanent contact with the skin of the user.

2. Structure according to claim 1, **characterized in that** the dimension of the outer element (1) measured perpendicular to the longitudinal XX axis is chosen to be less than the dimension of the intermediate element (3) at the level of the crotch of the wearer of so that said outer element (1) is not in contact with the pubic fold of the wearer.

3. Structure according to one of the preceding claims, **characterized in that** said edge (31) has, in the stretched and flattened state, a convexity turned towards the longitudinal XX axis.

4. Structure according to any one of the preceding claims, **characterized in that** the maximum distance H, which is measured perpendicular to the longitudinal XX axis, between the elastic border (31) and the longitudinal edge of the absorbent element is greater than 3 cm.

5. Structure according to any one of the preceding claims, **characterized in that** the outer element (1) is reusable, preferably washable and elastic.

6. Structure according to any one of the preceding claims, **characterized in that** the absorbent element (2) is reusable, preferably washable.

7. Structure according to any one of the preceding claims, **characterized in that** the attachment means (33) are arranged along the width of the absorbent element (2) and comprise reversible attachment elements.

8. Structure according to any one of claims 1 to 6, **characterized in that** the attachment means (33) are arranged along the width of the absorbent element and comprise non-removable attachment elements.

9. Structure according to any one of the preceding claims, **characterized in that** the outer element (1) has a general X shape and comprises an axis of symmetry X'X' which coincides with the longitudinal XX axis.

10. Structure according to any one of claims 1 to 9, **characterized in that** the outer element (1) has at least one non-extensible mechanical reinforcement zone in dorsal contact with the user, symmetrical with respect to the XX axis, and butterfly shaped.

11. Structure according to any one of the preceding claims, **characterized in that** it further comprises a disposable sleeve, covering at least the surface of the absorbent element (2) in contact with the skin of the user.

12. Structure according to any one of the preceding claims, **characterized in that** the intermediate element (3) further comprises longitudinal fins (35) linked to each of its elastic longitudinal borders (31).
